# EUROPÄISCHE PATENTSCHRIFT

(11) **EP 3 013 784 B1**
(45) Veröffentlichungstag und Bekanntmachung des Hinweises auf die Patenterteilung: **26.12.2018**
(21) Anmeldenummer: 14818631.5
(22) Anmeldetag: 16.06.2014
(51) Int. Cl.: C07C 51/265, C07C 51/31, C07C 63/16, C07D 307/89

(54) **VERFAHREN ZUM ANFAHREN EINES GASPHASENOXIDATIONSREAKTORS**
PROCESS FOR STARTING UP A GAS PHASE OXIDATION REACTOR
PROCÉDÉ DE DÉMARRAGE D'UN RÉACTEUR D'OXYDATION EN PHASE GAZEUSE

(30) Priorität: 26.06.2013 EP 13173691
(43) Veröffentlichungstag der Anmeldung: 04.05.2016
(73) Patentinhaber: BASF SE, 67056 Ludwigshafen am Rhein (DE)
(72) Erfinder: KRÄMER, Michael, 67734 Katzweiler (DE); FISCHER, Nico Frederik, 69115 Heidelberg (DE); ZÜHLKE, Jürgen, 67346 Speyer (DE); ALLMANN, Hans-Martin, 74867 Neunkirchen (DE)
(74) Vertreter: BASF IP Association
(86) Internationale Anmeldenummer: PCT/IB2014/062256
(87) Internationale Veröffentlichungsnummer: WO 2014/207604

(56) Entgegenhaltungen:
- WO-A1-02/16300
- WO-A1-2007/003662
- WO-A1-2009/124946
- WO-A1-2009/124947
- WO-A1-2011/128814

## Beschreibung

Die vorliegende Erfindung betrifft ein Verfahren zur Herstellung von Carbonsäuren und/oder Carbonsäureanhydriden durch Gasphasenoxidation von aromatischen Kohlenwasserstoffen.

Eine Vielzahl von Carbonsäuren und/oder Carbonsäureanhydriden wird technisch durch die katalytische Gasphasenoxidation von Kohlenwasserstoffen wie Benzol, den Xylolen, Naphthalin, Toluol oder Durol in Festbettreaktoren hergestellt. Man kann auf diese Weise z. B. Benzoesäure, Maleinsäureanhydrid, Phthalsäureanhydrid (PSA), Isophthalsäure, Terephthalsäure oder Pyromellithsäureanhydrid erhalten. Im Allgemeinen leitet man ein Gemisch aus einem sauerstoffhaltigen Gas und dem zu oxidierenden Ausgangsmaterial durch einen Reaktor mit einer Vielzahl von Rohren, in denen sich eine Schüttung eines Katalysators befindet. Zur Temperaturregelung sind die Rohre in der Regel von einem Wärmeträgermedium, beispielsweise einer Salzschmelze, umgeben.

Trotz dieser Thermostatisierung kann es aufgrund der hohen Exothermie der Umsetzungen innerhalb der Katalysatorschüttung zur Ausbildung von so genannten "Heißen Flecken" (Hot Spots) kommen, in denen die Temperatur wesentlich höher ist als in der übrigen Schüttung. Diese "Hot Spots" geben Anlass zu unerwünschten Nebenreaktionen wie beispielsweise der Totaloxidation des Ausgangsmaterials oder führen zur Bildung unerwünschter Nebenprodukte. Daneben besteht die Gefahr, dass der Katalysator - je nach Temperatur im Hot Spot - irreversibel geschädigt wird.

Zur Abschwächung dieser Hot Spots wurde in der Technik dazu übergegangen, unterschiedlich aktive Katalysatoren schichtweise in der Katalysatorschüttung anzuordnen. Die Katalysatoren können beispielsweise so angeordnet sein, dass der weniger aktive Katalysator zuerst mit dem Reaktionsgemisch in Kontakt kommt, d.h. er liegt in der Schüttung zum Gaseintritt hin, wohingegen sich der aktivere Katalysator zum Gasaustritt hin befindet (EP 163 231, EP 25 46 268). DE 10 2005 009 473 beschreibt einen Mehrlagenkatalysator zur Herstellung von PSA, bei dem die erste zur Gaseintrittsseite hin gelegene Katalysatorlage eine höhere Katalysatoraktivität besitzt als die zweite Katalysatorlage. Die Aktivität der folgenden Lagen steigt zur Gasautrittsseite, d.h. die dritte Katalysatorlage verfügt über eine höhere Aktivität als die zweite. WO2006/053732 beschreibt eine Katalysatorstrukturierung, bei der die Katalysatoraktivität der einzelnen Lagen von der Gaseintrittsseite zur Gasaustrittsseite ansteigt. Ferner wird die Möglichkeit beschrieben, dass innerhalb dieser Strukturierungen einzelne Lagen durch aktivere Vor- bzw. Zwischenlagen unterbrochen werden. Aktivitätsstrukturierungen, bei denen die unmittelbar an der Gaseintrittssseite befindliche Katalysatorlage (d.h. die erste Katalysatorlage) die am wenigsten aktive Lage ist, auf welche eine zweite aktivere Katalysatorlage folgt, auf die wiederum eine weniger aktive dritte Katalysatorlage (mit einer Aktivität zwischen der der ersten Lage und der der zweiten Lage) folgt und bei denen die Aktivität der möglicherweise noch folgenden Katalysatorlage(n) wieder steigt, sind demnach vorstellbar.

Zur Inbetriebnahme ("Anfahren") eines Katalysators wird die Katalysatorschüttung üblicherweise durch externe Beheizung des Reaktors auf eine Temperatur gebracht, die oberhalb der späteren Betriebstemperatur liegt. Der Katalysator wird in der Regel für mehrere Stunden bei dieser Temperatur in einem Gasstrom, der im Allgemeinen Sauerstoff enthält, präformiert (z.B. WO2006/131480).

Bevorzugt wird der Reaktor bei einem Luftstrom von 0.5 bis 1 Nm³/h/Rohr auf 100°C aufgeheizt. Zwischen 100°C und 250°C ist die Zersetzung des organischen Binders in dem Katalysator (WO2005/11862) zu erwarten und besonders bevorzugt wird der Luftstrom pro Rohr in diesem Temperaturbereich erhöht. Die thermische Zersetzung des Binders kann durch Analyse des Reaktorausgangstromes mit (wie in EP1831147, EP2024351 und EP2027102 beschrieben) oder ohne Nachreaktor verfolgt werden. Das früher übliche Ableiten der Zersetzungsprodukte nach dem Reaktor oder dem Nachreaktor in die Umwelt kann zur Verbesserung des Umweltschutzes entfallen, wenn die Möglichkeit der Abgasbehandlung besteht. Mögliche Ausführungsformen dieser Abgasbehandlung sind eine Abgaswäsche verbunden mit einer thermischen oder katalytischen Abgasverbrennung oder eine direkte Abgasverbrennung. Bei Temperaturen über 250°C bis zur Maximaltemperatur von z.B. 400°C wird der Luftstrom pro Rohr bevorzugt auf 0.1 bis 1 Nm³/h/Rohr abgesenkt. Bei der Maximaltemperatur wird der Reaktor bevorzugt für 12 bis 48 h belassen bevor die Temperatur zur eigentlichen Starttemperatur abgesenkt wird.

Sobald die eigentliche Oxidationsreaktion durch das Einleiten der aromatischen Kohlenwasserstoffe in den Gasstrom beginnt, wird die Reaktionstemperatur durch die ausgeprägte Exothermie der Umsetzung aufrecht erhalten und die externe Beheizung kann allmählich reduziert und schließlich abgestellt werden. Die Ausbildung eines ausgeprägten Hot Spots behindert allerdings eine rasche Erhöhung der Gasstrombeladung, da der Katalysator ab einer bestimmten Hot Spot-Temperatur irreversibel geschädigt wird. Daher wird die Beladung des Gasstroms mit dem zu oxidierenden Kohlenwasserstoff in kleinen Schritten erhöht während gleichzeitig die Temperatur des Wärmeträgermediums langsam reduziert wird, um die absolute Höhe de Hot Spot-Temperatur möglichst niedrig zu halten.

Unter der Anfahrzeit versteht man im Allgemeinen die Zeitspanne von der ersten Beaufschlagung eines Katalysators mit dem Edukt bis zum Erreichen eines im Wesentlichen stationären Produktionszustands. In der Regel soll die Anfahrzeit maximal 10% der Lebensdauer des Katalysators betragen, welche im Bereich mehrerer Jahre liegen kann (vgl. WO 2011/128814). Gemäß EP 2280921 liegt die Anfahrzeit beispielsweise bei einem PSA-Katalysator in einem Bereich von 2 bis 8 Wochen oder länger.

Aus dem Stand der Technik sind einige Anfahrprozeduren bekannt. So beschreibt DE 22 12 947A ein Verfahren zur Herstellung von PSA, bei dem man das Salzbad zu Beginn auf 370 bis 410°C temperiert und eine Menge an Luft von mindestens 1 Nm³/h mit einer Beladung von mindestens 33 g o-Xylol/Nm³ durch ein Reaktorrohr leitet, so dass sich eine Hot Spot-Temperatur von 450 bis 465 °C im ersten Drittel der Schüttung ausbildet.

DE 198 24 532 offenbart ein Verfahren zur Herstellung von PSA, bei dem die Beladung des Gasstroms bei einem konstanten Volumenfluss von 4 Nm³/h innerhalb mehrerer Tage von 40 g/Nm³ auf 80 g/Nm³ o-Xylol angehoben wurde.

DE 10 2005 031 465 offenbart ein Verfahren, bei dem die Katalysatorschüttung zwischen 360 und 450°C mit einem Luftstrom von 1 bis 3,5 Nm³/h und einer Beladung an o-Xylol von 20 bis 65 g/Nm³ angefahren wird. Dabei bildet sich in den ersten 7 bis 20 % der Katalysatorschüttung eine Hot spot-Temperatur von 360 bis kleiner 450°C aus.

EP 22080921 beschreibt ein Verfahren zum Anfahren eines Gasphasenoxidationsreaktors zur Oxidation von o-Xylol zu Phthalsäureanhydrid über einem Mehrlagenkatalysator, wobei die Anfangsbeladung wenigstens 30 g/Nm³ niedriger ist als die Zielbeladung und die Anfangstemperatur wenigstens 30°C höher ist als die Zieltemperatur. Die Zielbeladung wird mit 60 bis 110 g/Nm³ und die Zieltemperatur mit 340 bis 365°C angegeben.

DE10 2010 006 854 beschreibt ein Verfahren, bei dem die Katalysatorschüttung zunächst auf 365 bis 395 °C temperiert wird und mit einem Luftstrom von 0,5 bis 5 Nm³/h beaufschlagt wird, wobei der Luftstrom eine Kohlenwasserstoffbeladung von 10 bis 110 g/Nm³ besitzt. Dabei bildet sich ein Hot Spot in den letzten 10 bis 25 % der Katalysatorschüttung aus.

Um möglichst rasch die volle Leistungsfähigkeit eines Katalysators nutzen zu können, sollte die Anfahrzeit möglichst kurz sein. Neben einer Limitierung der Beladungssteigerung pro Zeiteinheit zur Begrenzung der Hot Spot-Temperatur in der Katalysatorschüttung spielt aber in der Anfahrzeit auch schon die Produktivität (d.h. hergestelltes Produkt/Zeiteinheit) und die Produktqualität eine wichtige Rolle. Die Oxidation von beispielsweise o-Xylol zu PSA ist eine Konsekutivreaktion, die über verschiedene Zwischenprodukte wie beispielsweise o-Toluylaldehyd und Phthalid verläuft (Krajewski et al., React. Kinet. Catal. Lett., 1977, Bd. 6, Seiten 461 bis 466). Diese Zwischenprodukte müssen ebenso wie das Edukt o-Xylol im Reaktionsverlauf möglichst quantitativ umgesetzt werden. Im Reaktoraustrittsgas werden Gehalte an o-Xylol und Phthalid von jeweils < 0,1 Gew.-% angestrebt.

Gemäß DE 10 20120 006 854 kann einer Verschlechterung der Produktqualität durch Erhöhung der Reaktionstemperatur, beispielsweise durch (a) Erhöhung der Wärmebetttemperatur, oder (b) durch Verringerung der Luftmenge bei gleichbleibender Kohlenwasserstoffbeladung entgegengewirkt werden. Dies geht jedoch mit einem Rückgang der Produktmenge einher, da im Falle (a) die Selektivität fällt und im Falle (b) eine geringere Menge an Edukt ein- und somit auch umgesetzt wird.

Die Leistungsfähigkeit der Verfahren zur Herstellung von Carbonsäuren und/oder Carbonsäureanhydriden durch Gasphasenoxidation von aromatischen Kohlenwasserstoffen bemisst sich in erster Linie an den erhaltenen Ausbeuten sowie an deren Reinheit. Beispielsweise zeichnet sich qualitativ hochwertiges PSA (vgl. DE 10 2010 006 854 A1, WO 2011/128814 A1) durch möglichst niedrige Gehalte sowohl an Unteroxidationsprodukten (insbesondere Phthalid bzw. Naphthochinon) als auch an nicht umgesetztem o-Xylol bzw. Naphthalin aus, da diese Stoffe sich nur sehr schwer voneinander trennen lassen und die Farbzahl des fertigen rein-PSAs negativ beeinflussen.

Der Einfluss von An- und Abfahrprozessen auf die Stabilität von Katalysatoren für die Oxidation von o-Xylol zu PSA im Modellversuch wurde untersucht und von H. Bo et al. in Petrochemical Technology 2004, Bd. 5, Seiten 421 bis 423 beschrieben.

In WO 2009/124947 A1 wird erläutert, dass Verfahren bekannt sind, bei denen während des Anfahrens der Gasphasenoxidation aromatischer Kohlenwasserstoffe die Temperatur des Wärmeträgermediums abgesenkt wird, während parallel dazu die Beladung auf Ziellast erhöht wird.

Es besteht ein ständiger Bedarf nach verbesserten Verfahren für Gasphasenoxidationen, die einen möglichst hohen Umsatz bei hoher Selektivität und Produktreinheit ermöglichen.

Der vorliegenden Erfindung lag die Aufgabe zugrunde, ein Verfahren zur Herstellung von Carbonsäuren und/oder Carbonsäureanhydriden durch Gasphasenoxidation von aromatischen Kohlenwasserstoffen bereitzustellen, welches bei hohem Umsatz und gleichzeitig hoher Selektivität Zugang zu möglichst reinen Produkten ermöglicht.

Diese Aufgabe wird gelöst durch ein Verfahren zur Herstellung von Carbonsäuren und/oder Carbonsäureanhydriden durch Gasphasenoxidation von aromatischen Kohlenwasserstoffen, bei dem man bei gleicher bzw. nahezu gleicher Kohlenwasserstoffbeladung und gleichem bzw. nahezu gleichem Volumenfluss das kontinuierliche Absenken der Reaktionstemperatur für eine gewisse Zeit aussetzt.

Gegenstand der Erfindung ist somit ein Verfahren zur Herstellung von Carbonsäuren und/oder Carbonsäureanhydriden durch Gasphasenoxidation von aromatischen Kohlenwasserstoffen, bei dem man einen Gasstrom, der mindestens einen aromatischen Kohlenwasserstoff und molekularen Sauerstoff umfasst, kontinuierlich über einen durch ein Wärmeträgermedium thermostatisierten Katalysator leitet, dadurch gekennzeichnet, dass man die Temperatur des Wärmeträgermediums während des Anfahrens des Reaktors von einer Temperatur im Bereich von 380 bis 410 °C auf eine Temperatur im Bereich von 340 bis 365 °C absenkt und dabei für mindestens 24 Stunden konstant hält mit einer Änderung von weniger als 0,1 °C pro 24 Stunden und währenddessen weder die Beladung des Gasstroms mit Kohlenwasserstoffen noch das Gasstromvolumen um mehr als 3 % erhöht.

In einer bevorzugten Ausführungsform der Erfindung wird in dem Zeitraum, während dessen die Temperatur des Wärmeträgermediums konstant gehalten wird, die Beladung des Gasstroms mit Kohlenwasserstoffen um maximal 1,5 % erhöht.

In einer weiteren bevorzugten Ausführungsform der Erfindung wird in dem Zeitraum, während dessen die Temperatur des Wärmeträgermediums konstant gehalten wird, das Gasstromvolumen um maximal 2,5 % erhöht.

Bei der Herstellung von Carbonsäuren und/oder Carbonsäureanhydriden durch Gasphasenoxidation von aromatischen Kohlenwasserstoffen wird das den Reaktor umgebende Wärmeträgermedium zu Beginn des Anfahrens üblicherweise auf eine Temperatur von 380 bis 410°C erwärmt, die dann allmählich auf eine Temperatur von 340 bis 365°C abgesenkt wird während gleichzeitig die Beladung des Gasstroms mit Kohlenwasserstoffen von etwa 25 bis 30 g/Nm³ auf etwa 70 bis 120 g/Nm³ ansteigt. Dabei kann man die Beladung des Gasstroms mit Kohlenwasserstoffen beispielsweise mit einer Rate von 0,5 bis 10 g/Nm³ pro Tag erhöhen.

Im Allgemeinen ist die Anfangstemperatur wenigstens 30 °C höher ist als die Betriebstemperatur, meist 35 bis 50 °C höher als die Betriebstemperatur. Unter Betriebstemperatur wird die Temperatur des Wärmeträgermediums im stationären Zustand, d. h. nach beendeter Anfahrphase während des produktiven Betriebs des Reaktors angesehen. Zur Kompensation nachlassender Katalysatoraktivität kann man im Betriebszustand die Temperatur des Wärmeträgermediums allerdings langfristig leicht erhöhen (um weniger als 10°C/Jahr).
Unter allmählichem Absenken der Temperatur des Wärmeträgermediums versteht man im Rahmen dieser Erfindung eine messbare Erniedrigung der Temperatur des Wärmeträgermediums um mindestens 0,1°C pro 24 Stunden. Unter Konstanthalten der Temperatur des Wärmeträgermediums versteht man im Rahmen dieser Erfindung, dass sich die Temperatur des Wärmeträgermediums um weniger als 0,1°C pro 24 Stunden ändert.

Das beim erfindungsgemäßen Verfahren verwendete Gastromvolumen richtet sich nach der Reaktorgröße und der für die jeweilige Umsetzung erforderlichen Verweilzeit und liegt üblicherweise im Bereich von 0,5 bis 5 Nm³/h pro Reaktorrohr, bevorzugt im Bereich von 2,5 bis 4,2 Nm³/h.

Das erfindungsgemäße Verfahren eignet sich beispielsweise zur Gasphasenoxidation aromatischer C₆- bis C₁₀-Kohlenwasserstoffe wie Benzol, den Xylolen, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhyrid. Das Verfahren eignet sich besonders zur Herstellung von Phthalsäureanhydrid aus o-Xylol und/oder Naphthalin. Die Gasphasenreaktionen zur Herstellung von Phthalsäureanhydrid sind allgemein bekannt und beispielsweise in WO 2004/103561 auf der Seite 6 beschrieben.

Als Katalysatoren haben sich für diese Oxidationsreaktionen sogenannte Schalenkatalysatoren bewährt, bei denen die katalytisch aktive Masse schalenförmig auf einem inerten Trägermaterial, wie Steatit, aufgebracht ist. Als katalytisch aktive Bestandteile der katalytisch aktiven Masse dieser Schalenkatalysatoren werden im Allgemeinen Titandioxid und Vanadiumpentoxid verwendet. Des Weiteren können in der katalytisch aktiven Masse in geringen Mengen eine Vielzahl anderer oxidischer Verbindungen enthalten sein, die als Promotoren die Aktivität und Selektivität des Katalysators beeinflussen.

Als inertes Trägermaterial können praktisch alle Trägermaterialien des Standes der Technik, wie sie vorteilhaft bei der Herstellung von Schalenkatalysatoren für die Oxidation von aromatischer Kohlenwasserstoffe zu Aldehyden, Carbonsäuren und /oder Carbonsäureanhydriden eingesetzt werden, Verwendung finden, wie beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Die Katalysatorträger können beispielsweise in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenreaktionen von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 3 bis 8 mm und einer Wandstärke von 1 bis 2 mm verwendet.

Für die katalytische Gasphasenoxidation von o-Xylol und/oder Naphthalin zu PSA geeignete Katalysatoren enthalten eine katalytisch aktive Masse, welche mindestens Vanadiumoxid und Titandioxid umfasst und in einer oder mehreren Schichten auf das Trägermaterial aufgebracht werden kann. Verschiedene Schichten können sich dabei in ihrer Zusammensetzung unterscheiden.

Vorzugsweise enthält die katalytisch aktive Masse, bezogen auf die Gesamtmenge der katalytisch aktiven Masse, 1 bis 40 Gew.-% Vanadiumoxid, berechnet als V₂O₅, und 60 bis 99 Gew.-% Titandoxid, berechnet als TiO₂. Die katalytisch aktive Masse kann in bevorzugten Ausführungsformen daneben bis zu 1 Gew.-% einer Cäsiumverbindung, berechnet als Cs, bis zu 1 Gew.-% einer Phosphorverbindung, berechnet als P, und bis zu 10 Gew.-% Antimonoxid, berechnet als Sb₂O₃ enthalten. Alle Angaben zur Zusammensetzung der katalytisch aktiven Masse beziehen sich auf deren kalzinierten Zustand, z. B. nach Kalzinierung des Katalysators für eine Stunde bei 450°C.

Üblicherweise wird Titandioxid in der Anatas-Form für katalytisch aktive Masse verwendet. Das Titandioxid weist vorzugsweise eine BET-Oberfläche von 15 bis 60 m²/g, insbesondere 15 bis 45 m²/g, besonders bevorzugt 13 bis 28 m²/g auf. Das eingesetzte Titandioxid kann aus einem einzelnen Titandioxid oder einem Gemisch von Titandioxiden bestehen. In letzterem Fall bestimmt sich der Wert der BET-Oberfläche als gewichteter Mittelwert der Beiträge der einzelnen Titandioxide. Das eingesetzte Titandioxid besteht z. B. vorteilhaft aus einem Gemisch eines TiO₂ mit einer BET-Oberfläche von 5 bis 15 m²/g und eines TiO₂ mit einer BET-Oberfläche von 15 bis 50 m²/g.

Als Vanadiumquelle eignen sich besonders Vanadiumpentoxid oder Ammoniummetavanadat. Als Antimonquelle eignen sich verschiedene Antimonoxide, insbesondere Antimontrioxid. Vanadium und Antimon können darüber hinaus auch in Form einer Vanadiumantimonatverbindung eingesetzt werden (WO 2011/061132). Das in der Aktivmasse mindestens einer Lage eingebrachte Vanadiumantimonat kann durch Umsetzung beliebiger Vanadium- und Antimonverbindungen hergestellt werden. Bevorzugt ist die direkte Umsetzung der Oxide zu einem Mischoxid bzw. Vanadiumantimonat. Das Vanadiumantimonat kann unterschiedliche molare Verhältnisse von Vanadium zu Antimon aufweisen sowie ggf. auch weitere Vanadium- bzw. Antimonverbindungen enthalten und in Mischung mit weiteren Vanadium- bzw. Antimonverbindungen eingesetzt werden.

Als Phosphorquelle kommen insbesondere Phosphorsäure, phosphorige Säure, hypophosphorige Säure, Ammoniumphosphat oder Phosphorsäureester und vor allem Ammoniumdihydrogenphosphat in Betracht. Als Quellen von Cäsium kommen das Oxide oder Hydroxid oder die thermisch in das Oxid überführbare Salze wie Carboxylate, insbesondere das Acetat, Malonat oder Oxalat, Carbonat, Hydrogencarbonat, Sulfat oder Nitrat in Betracht.

Neben den optionalen Zusätzen Cäsium und Phosphor können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Als solche Promotoren seien beispielhaft die Alkalimetalle, insbesondere außer dem genannten Cäsium, Lithium, Kalium und Rubidium, die meist in Form ihrer Oxide oder Hydroxide eingesetzt werden, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid genannt.

Ferner kommen von den genannten Promotoren noch bevorzugt als Zusätze die Oxide von Niob und Wolfram in Mengen von 0,01 bis 0,50 Gew.-%, bezogen auf die katalytisch wirksame Masse in Betracht.

Das Aufbringen der Schicht(en) des Schalenkatalysators erfolgt zweckmäßigerweise durch Aufsprühen einer Suspension von TiO₂ und V₂O₅, die gegebenenfalls Quellen der oben genannten Promotorelemente enthält, auf den fluidisierten Träger (EP 1670741, WO 2005/030388). Vor der Beschichtung wird die Suspension vorzugsweise ausreichend lange, z. B. 2 bis 30 Stunden, insbesondere 12 bis 25 Stunden, gerührt, um Agglomerate der suspendierten Feststoffe aufzubrechen und eine homogene Suspension zu erhalten. Die Suspension hat typischerweise einen Feststoffgehalt von 20 bis 50 Gew.-%. Das Suspensionsmedium ist im Allgemeinen wässrig, z. B. Wasser selbst oder ein wässriges Gemisch mit einem wassermischbaren organischen Lösungsmittel, wie Methanol, Ethanol, Isopropanol, Formamid und dergleichen.

In der Regel werden der Suspension organische Binder, bevorzugt Copolymere, vorteilhaft in Form einer wässrigen Dispersion, von Acrylsäure/Maleinsäure, Vinylacetat/Vinyllaurat, Vinylacetat/Acrylat, Styrol/Acrylat sowie Vinylacetat/Ethylen zugesetzt. Die Binder sind als wässrige Dispersionen handelsüblich, mit einem Feststoffgehalt von z. B. 35 bis 65 Gew.-%. Die eingesetzte Menge solcher Binderdispersionen beträgt im Allgemeinen 2 bis 45 Gew.-%, vorzugsweise 5 bis 35 Gew.-%, besonders bevorzugt 7 bis 20 Gew.-%, bezogen auf das Gewicht der Suspension.

Der Träger wird in z. B. einer Wirbelschicht- bzw. Fließbettapparatur in einem aufsteigenden Gasstrom, insbesondere Luft, fluidisiert. Die Apparate bestehen meist aus einem konischen oder kugelförmigen Behälter, bei dem das fluidisierende Gas von unten oder von oben über ein Tauchrohr eingeführt wird. Die Suspension wird über Düsen von oben, seitlich oder von unten in die Wirbelschicht eingesprüht. Vorteilhaft ist der Einsatz eines mittig bzw. konzentrisch um das Tauchrohr angeordneten Steigrohrs. Innerhalb des Steigrohres herrscht eine höhere Gasgeschwindigkeit, die die Trägerpartikel nach oben transportiert. Im äußeren Ring liegt die Gasgeschwindigkeit nur wenig oberhalb der Lockerungsgeschwindigkeit. So werden die Partikel kreisförmig vertikal bewegt. Eine geeignete Fließbettvorrichtung ist z. B. in der DE-A 4006935 beschrieben.
Bei der Beschichtung des Katalysatorträgers mit der katalytisch aktiven Masse werden im Allgemeinen Beschichtungstemperaturen von 20 bis 500°C angewandt, wobei die Beschichtung unter Atmosphärendruck oder unter reduziertem Druck erfolgen kann. Im Allgemeinen erfolgt die Beschichtung bei 0°C bis 200°C, vorzugsweise bei 20 bis 150°C, insbesondere bei 60 bis 120°C durchgeführt.

Die Schichtdicke der katalytisch aktiven Masse beträgt in der Regel 0,02 bis 0,2 mm, vorzugsweise 0,05 bis 0,15 mm. Der Aktivmasseanteil am Katalysator beträgt üblicherweise 5 bis 25 Gew.-%, meist 7 bis 15 Gew.-%.

Durch thermische Behandlung des so erhaltenen Präkatalysators bei Temperaturen über 200 bis 500°C entweicht das Bindemittel durch thermische Zersetzung und/oder Verbrennung aus der aufgetragenen Schicht. Vorzugsweise erfolgt die thermische Behandlung in situ im Gasphasenoxidationsreaktor.

Es hat sich als besonders vorteilhaft erwiesen, wenn eine Katalysatorschüttung aus verschiedenen Katalysatoren eingesetzt wird, die sich in ihrer katalytischen Aktivität und/oder der chemischen Zusammensetzung ihrer Aktivmasse unterscheiden und die nacheinander als Lagen in unterschiedliche Zonen des Reaktors eingefüllt werden. Vorzugsweise wird bei Anwendung zweier Reaktionszonen in der ersten, also zum Eintritt des Gasstroms hin gelegenen Reaktionszone, ein Katalysator eingesetzt, der in Vergleich zum Katalysator, welcher sich in der zweiten, also zum Austritt des Gasstroms hin gelegenen Reaktionszone, befindet, eine etwas geringere katalytische Aktivität aufweist. Im Allgemeinen wird die Umsetzung durch die Temperatureinstellung so gesteuert, dass in der ersten Zone der größte Teil der im Gasstrom enthaltenen aromatischen Kohlenwasserstoffe bei maximaler Ausbeute umgesetzt wird. Bevorzugt werden drei- bis fünflagige Katalysatorsysteme verwendet, insbesondere drei- und vierlagige Katalysatorsysteme. Ein dreilagiges Katalysatorsystem für die o-Xyloloxidation zu PSA ist beispielsweise in EP 1084115 beschrieben.

Anstelle gegeneinander abgegrenzter Lagen der verschiedenen Katalysatoren kann auch ein quasi-kontinuierlicher Übergang der Lagen und damit eine quasi gleichmäßige Änderung der Aktivmassezusammensetzung bzw. ihres Gehalts dadurch bewirkt werden, dass man beim Übergang von einer Lage zur nächsten Lage eine Zone mit einer Vermischung der aufeinander folgenden Katalysatoren einfügt.

Die Schüttungslänge der ersten Katalysatorlage (KL1) liegt vorzugsweise im Bereich von 10 bis 50 %, besonders bevorzugt im Bereich von 15 bis 30 % der gesamten Katalysatorfüllhöhe im Reaktor. Typische Reaktoren weisen eine Füllhöhe von 250 cm bis 400 cm auf. Die Katalysatorlagen können gegebenenfalls auch auf mehrere Reaktoren verteilt werden.

Durch das erfindungsgemäße Verfahren wird eine deutliche Verbesserung der Produktqualität erreicht, ausgedrückt in reduzierten Gehalten an nicht umgesetztem aromatischem Kohlenwasserstoff und auch an Unteroxidationsprodukten wie z. B. Phthalid oder Naphthochinon im Falle der Herstellung von PSA aus o-Xylol und/oder Naphthalin.

Das erfindungsgemäße Verfahren kann auch bei katalytischen Gasphasenoxidationen zur Verbesserung der Produktqualität eingesetzt werden.

Ein weiterer Gegenstand der Erfindung ist die Verwendung des oben beschriebenen Verfahrens zur Verbesserung der Produktqualität bei der Herstellung von Carbonsäuren und/oder Carbonsäureanhydriden durch Gasphasenoxidation von aromatischen Kohlenwasserstoffen.

Die nachfolgenden Beispiele erläutern die Erfindung ohne sie zu begrenzen.

### Beispiele A: Oxidation von o-Xylol zu Phthalsäureanhydrid

### Herstellung der Katalysatorlagen

### Katalysatorlage 1 (KL1) (Vanadiumantimonat als V- und Sb-Quelle):

### Herstellung des Vanadiumantimonats:

2284,1 g Vanadiumpentoxid und 1462 g Antimontrioxid (Antraco ACC-BS, ca. 4 % Valentinit und 96 % Senarmontit; Sb₂O₃ ≥ 99,8 Gew.-%; As ≤ 800 Gew.-ppm, Pb ≤ 800 Gew.-ppm, Fe ≤ 30 Gew.-ppm, mittlere Partikelgröße = 1,4 µm) wurden in 5,6 l demineralisiertem Wasser suspendiert und die Suspension 15 Stunden unter Rückfluss gerührt. Im Anschluss wurde die Suspension auf 80°C abgekühlt und mittels Sprühtrocknung getrocknet. Die Eingangstemperatur lag dabei bei 340°C, die Austrittstemperatur bei 110°C. Das so erhaltene Sprühpulver hatte eine BET-Oberfläche von 89 m²/g und wies einen Gehalt an Vanadium von 32 Gew.-% sowie einen Gehalt an Antimon von 30 Gew.-% auf. Das Produkt wies folgende kristalline Bestandteile auf: Valentinit (ICPDS: 11-0689): ca. 3%; Senarmontit (ICPDS: 43-1071): ca. 2%; Vanadiumantimonat (ICPDS: 81-1219): ca. 95%. Die mittlere Kristallitgröße des Vanadiumantimonates betrug ca. 9 nm.

### Suspensionsansatz und Beschichtung:

2 kg Steatitringe (Magnesiumsilikat) mit Abmessungen von 7 mm x 7 mm x 4 mm wurden in einer Fließbettapparatur mit 752 g einer Suspension aus 4,4 g Cäsiumcarbonat, 413,3 g Titandioxid (Fuji TA 100 CT; Anatas, BET-Oberfläche 27 m²/g), 222,5 g Titandioxid (Fuji TA 100; Anatas, BET-Oberfläche 7 m²/g), 86,9 g Vanadiumantimonat (wie oben hergestellt), 1870,1 g demineralisiertem Wasser und 76,7 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 8,3 %. Die analysierte Zusammensetzung der Aktivmasse bestand aus 7,1 % V₂O₅, 4,5 % Sb₂O₃, 0,50 % Cs, Rest TiO₂.

### Katalysatorlage 2 (KL2) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 920 g einer Suspension aus 3,0 g Cäsiumcarbonat, 446,9 g Titandioxid (Fuji TA 100 C; Anatas, BET-Oberfläche 20 m²/g), 133,5 g Titandioxid (Fuji TA 100; Anatas, BET-Oberfläche 7 m²/g), 45,4 g Vanadiumpentoxid, 11,6 g Antimontrioxid, 1660,1 g demineralisiertem Wasser und 104,5 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450°C betrug die auf die Steatitringe aufgebrachte Aktivmasse 10,0 %. Die analysierte Zusammensetzung der Aktivmasse bestand aus 7,1 % V₂O₅, 1,8 % Sb₂O₃, 0,38 % Cs, Rest TiO₂.

### Katalysatorlage 3 (KL3) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 750 g einer Suspension aus 2,33 g Cäsiumcarbonat, 468,7 g Titandioxid (Fuji TA 100 C; Anatas, BET-Oberfläche 20 m²/g), 76,3 g Titandioxid (Fuji TA 100; Anatas, BET-Oberfläche 7 m²/g), 48,7 g Vanadiumpentoxid, 16,7 g Antimontrioxid, 1588,0 g demineralisiertem Wasser und 85,2 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450°C betrug die auf die Steatitringe aufgebrachte Aktivmasse 8,5 %. Die analysierte Zusammensetzung der Aktivmasse bestand aus 7,95 % V₂O₅, 2,7 % Sb₂O₃, 0,31 % Cs, Rest TiO₂.

### Katalysatorlage 4 (KL4) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 760 g einer Suspension aus 1,7 g Cäsiumcarbonat, 370,1 g Titandioxid (Fuji TA 100 CT; Anatas, BET-Oberfläche 27 m²/g), 158,6 g Titandioxid (Fuji TA 100; Anatas, BET-Oberfläche 7 m²/g), 67,3 g Vanadiumpentoxid, 14,8 g Antimontrioxid, 1587,9 g demineralisiertem Wasser und 86,3 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450°C betrug die auf die Steatitringe aufgebrachte Aktivmasse 8,5 %. Die analysierte Zusammensetzung der Aktivmasse bestand aus 11 % V₂O₅, 2,4 % Sb₂O₃, 0,22 % Cs, Rest TiO₂.

### Katalysatorlage 5 (KL5) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 850 g einer Suspension aus 389,8 g Titandioxid (Fuji TA 100 CT; Anatas, BET-Oberfläche 27 m²/g), 97,5 g Titandioxid (Fuji TA 100; Anatas, BET-Oberfläche 7 m²/g), 122,4 g Vanadiumpentoxid, 1587,9 g demineralisiertem Wasser und 96,5 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450°C betrug die auf die Steatitringe aufgebrachte Aktivmasse 9,1 %. Die analysierte Zusammensetzung der Aktivmasse bestand aus 20 % V₂O₅, 0,38 % P, Rest TiO₂.
Beispiel 1 (erfindungsgemäß): Katalytische Oxidation von o-Xylol zu Phthalsäureanhydrid im großtechnischen Maßstab

Die katalytische Oxidation von o-Xylol zu Phthalsäureanhydrid wurde im technischen Maßstab in einem salzbadgekühlten Rohrbündelreaktor mit insgesamt 18900 Rohren durchgeführt. Vom Reaktoreingang zum Reaktorausgang wurden jeweils 80 cm KL1, 60 cm KL2, 70 cm KL3, 50 cm KL4 und 60 cm KL5 in die einzelnen Rohre mit einer lichten Weite von 25 mm eingefüllt. Die Katalysatorlagen wurden in Anlehnung an die oben beschriebene Herstellung der KL1 bis KL5 in Ansätzen von jeweils 150 kg Steatitringen hergestellt. Die Eisenrohre waren zur Temperaturregelung von einer Salzschmelze umgeben.

Präformierung des Katalysators erfolgte unter Luft bei 400°C.

Der Katalysator wurde bei 386°C angefahren und folgende Gasströme von oben nach unten (berechnet als Strom pro Einzelrohr) durch die Rohre geleitet: Luft 3,0 bis 4,0 Nm³/h Luft mit Beladungen an 98,8 bis 99,4 Gew.-%igem o-Xylol von 30 bis 100 g/Nm³ geleitet. Die Wärmebetttemperatur (Salzbadtemperatur) wurde ausgehend von 386°C abgesenkt und gleichzeitig die Beladung an o-Xylol gemäß der folgenden Tabelle variiert. Die PSA-Ausbeuten wurden im Reaktionsaustrittsgas gemessen und sind in Massen-% (kg PSA pro kg umgesetztem o-Xylol), bezogen auf 100 %-iges o-Xylol angeben.

| Laufzeit [Stunden] | Salzbadtemperatur [°C] | Gasstromvolumen [Nm³/h] | Beladung [g o-Xylol/Nm³] | o-Xylol im Reaktoraustritt [Gew.-%] | Phthalid im Reaktoraustritt [Gew.-%] |
|---|---|---|---|---|---|
| 24 | 386 | 3,0 | 35,0 | 0,002 | 0,009 |
| 48 | 382 | 3,25 | 37,0 | 0,002 | 0,008 |
| 72 | 380,1 | 3,5 | 38,5 | 0,003 | 0,009 |
| 96 | 378 | 3,75 | 40,0 | 0,004 | 0,021 |
| 120 | 376 | 3,8 | 40,5 | 0,003 | 0,009 |
| 144 | 374 | 3,8 | 43,5 | 0,004 | 0,014 |
| 168 | 373 | 3,8 | 45,0 | 0,004 | 0,014 |
| 192 | 372 | 3,85 | 46,0 | 0,014 | 0,031 |
| 216 | 371 | 3,88 | 47,0 | 0,004 | 0,019 |
| 240 | 370 | 3,83 | 48,0 | 0,005 | 0,022 |
| 264 | 369 | 3,83 | 49,0 | 0,007 | 0,022 |
| 288 | 368 | 3,83 | 50,0 | 0,007 | 0,025 |
| 312 | 367 | 3,83 | 51,0 | 0,007 | 0,026 |
| 336 | 366 | 3,83 | 53,0 | 0,008 | 0,028 |
| 360 | 365 | 3,83 | 54,8 | 0,007 | 0,031 |
| 384 | 363 | 3,83 | 56,0 | 0,009 | 0,035 |
| 408 | 361 | 3,83 | 57,4 | 0,010 | 0,041 |
| 432 | 359 | 3,83 | 59,2 | 0,014 | 0,062 |
| 456 | 358,5 | 3,83 | 60,9 | 0,026 | 0,059 |
| 480 | 357,5 | 3,83 | 62,7 | 0,030 | 0,064 |
| 504 | 356,6 | 3,85 | 63,7 | 0,031 | 0,066 |
| 528 | 355,5 | 3,85 | 65,0 | 0,043 | 0,083 |
| 552 | 354,5 | 3,85 | 66,0 | 0,068 | 0,102 |
| 576 | 354,4 | 3,85 | 67,0 | 0,088 | 0,123 |
| 600 | 354,4 | 3,85 | 67,1 | 0,079 | 0,100 |
| 624 | 354,4 | 3,85 | 68,0 | 0,060 | 0,092 |
| 648 | 353,9 | 3,85 | 69,3 | 0,067 | 0,100 |
| 672 | 353,9 | 3,85 | 70,0 | 0,060 | 0,090 |
| 696 | 353,4 | 3,88 | 71 | 0,069 | 0,098 |

Man kann erkennen, dass die Gehalte an o-Xylol und dem Unteroxidationsprodukt Phthalid im Reaktoraustrittsgas durch Aussetzen des Absenkens der Temperatur reduziert werden (nach 576 bis 624 h bzw. nach 648 bis 672 h), wobei in diesem Fall die Beladung an o-Xylol gleichzeitig leicht erhöht wurde.

### Beispiel 2 (erfindingsgemäß): Katalytische Oxidation von o-Xylol zu Phthalsäureanhydrid im Modellrohrmaßstab

Die katalytische Oxidation von o-Xylol zu Phthalsäureanhydrid wurde in einem salzbadgekühlten Rohrreaktor mit einem Innendurchmesser der Rohre von 25 mm durchgeführt. Von Reaktoreingang zu Reaktorausgang wurden jeweils 80 cm KL1, 60 cm KL2, 70 cm KL3, 50 cm KL4 und 60 cm KL5 in ein 3,5 m langes Eisenrohr mit der lichten Weite von 25 mm eingefüllt. Das Eisenrohr war zur Temperaturregelung von einer Salzschmelze umgeben, eine 4 mm Außendurchmesser Thermohülse mit eingebautem Zugelement diente der Katalysatortemperaturmessung.

Präformierung der Katalysatoren erfolgte unter 0,1 Nm³/h Luft bei 400°C für ca. 38 Stunden.

Nach Anfahren des Katalysators bei 386°C wurde das Rohr stündlich von oben nach unten mit 3,0 bis 4,0 Nm³ Luft mit Beladungen an 99-99,4 Gew.-%igem o-Xylol von 30 bis 100 g/Nm³ durchströmt. Die Wärmebetttemperatur (Salzbadtemperatur) wurde ausgehend von 386°C abgesenkt. Die PSA-Ausbeuten wurden im Reaktionsaustrittsgas gemessen und sind in Massen-% (kg PSA pro kg umgesetztem o-Xylol), bezogen auf 100 %-iges o-Xylol angeben.

Die unterschiedlichen Betriebsparameter sind in der folgenden Tabelle zusammengefasst:

| Laufzeit [Stunden] | Salzbadtemperatur [°C] | Gasstrom volumen [Nm³/h] | Beladung [g o-Xylol/Nm³] | o-Xylol im Reaktoraustritt [Gew.-%] | Phthalid im Reaktoraustritt [Gew.-%] |
|---|---|---|---|---|---|
| 24 | 386 | 3,3 | 33 | n.b. | n.b. |
| 48 | 382 | 3,3 | 33 | n.b. | n.b. |
| 72 | 380 | 3,3 | 33 | 0,00 | 0,05 |
| 96 | 378 | 3,5 | 43 | 0,01 | 0,06 |
| 120 | 376 | 3,7 | 45 | 0,02 | 0,09 |
| 144 | 374 | 4,0 | 46 | 0,04 | 0,14 |
| 168 | 372 | 4,0 | 48 | 0,05 | 0,16 |
| 192 | 370 | 4,0 | 51 | n.b. | n.b. |
| 216 | 368 | 4,0 | 51 | n.b. | n.b. |
| 240 | 366 | 4,0 | 51 | n.b. | n.b. |
| 264 | 365 | 4,0 | 51 | 0,12 | 0,23 |
| 288 | 365 | 4,1 | 51 | 0,11 | 0,23 |
| 312 | 365 | 4,0 | 51 | 0,08 | 0,19 |
| 336 | 367 | 4,0 | 51 | 0,06 | 0,10 |

| | | | | | |
|---|---|---|---|---|---|
| n.b.: nicht bestimmt | | | | | |

Man kann erkennen, dass die Gehalte an o-Xylol und dem Unteroxidationsprodukt Phthalid im Reaktoraustrittsgas durch Aussetzen des Absenkens der Temperatur reduziert werden (nach 288 bis 312 h), wobei in diesem Fall der Volumenstrom gleichzeitig leicht erhöht wurde.

Die folgenden Beispiele 3 bis 8 wurden analog zu Beispiel 1 und 2 bei 386°C angefahren und die Salzbadtemperatur kontinuierlich abgesenkt bis die dargestellten Betriebspunkte erreicht wurden.

### Beispiel 3 (erfindungsgemäß):

Die unterschiedlichen Betriebsparameter sind in der folgenden Tabelle zusammengefasst:

| Laufzeit [Stunden] | Salzbadtemperatur [°C] | Gasstromvolumen [Nm³/h] | Beladung [g o-Xylol/Nm³] | o-Xylol im Reaktoraustritt [Gew.-%] | Phthalid im Reaktoraustritt [Gew.-%] | PSA-Ausbeute [Gew.-%] |
|---|---|---|---|---|---|---|
| 192 | 368 | 4 | 63 | 0,07 | 0,19 | 112,3 |
| 216 | 366 | 4 | 66 | 0,11 | 0,23 | 112,0 |
| 240 | 365 | 4 | 68 | 0,12 | 0,24 | 112,2 |
| 264 | 365 | 4 | 68 | 0,10 | 0,22 | 112,4 |
| 288 | 365 | 4 | 68 | 0,09 | 0,19 | 112,3 |

### Beispiel 4 (erfindungsgemäß):

Die unterschiedlichen Betriebsparameter sind in der folgenden Tabelle zusammengefasst:

| Laufzeit [Tage] | Salzbadtemperatur [°C] | Gasstromvolumen [Nm³/h] | Beladung [g o-Xylol/Nm³] | o-Xylol im Reaktoraustritt [Gew.-%] | Phthalid im Reaktoraustritt [Gew.-%] | PSA-Ausbeute [Gew.-%] |
|---|---|---|---|---|---|---|
| 192 | 370 | 4,0 | 67 | 0,09 | 0,22 | 111,7 |
| 216 | 368 | 4,0 | 71 | 0,11 | 0,24 | 111,8 |
| 240 | 368 | 4,0 | 71 | 0,08 | 0,21 | 111,9 |

Ein Aussetzen des Absenkens der Salzbadtemperatur in den Beispielen 3 und 4 führte zu einer Verbesserung der Produktgaszusammensetzung, wobei die sonstigen Reaktionsparameter unverändert blieben.

### Beispiel 5 (nicht erfindungsgemäß):

Die unterschiedlichen Betriebsparameter sind in der folgenden Tabelle zusammengefasst:

| Laufzeit [Tage] | Salzbadtemperatur [°C] | Gasstromvolumen [Nm³/h] | Beladung [g o-Xylol/Nm³] | o-Xylol im Reaktoraustritt [Gew.-%] | Phthalid im Reaktoraustritt [Gew.-%] | PSA-Ausbeute [Gew.-%] |
|---|---|---|---|---|---|---|
| 240 | 368 | 4,0 | 54 | 0,05 | 0,14 | 109,7 |
| 264 | 366 | 4,0 | 57 | 0,08 | 0,16 | 111,2 |
| 288 | 365 | 4,0 | 60 | 0,12 | 0,18 | 110,8 |
| 312 | 364 | 4,0 | 60 | n.d. | n.d. | n.d. |
| 336 | 363 | 4,0 | 62 | 0,13 | 0,19 | 110,5 |
| 360 | 362 | 4,0 | 64 | n.d. | n.d. | n.d. |
| 384 | 361,3 | 4,0 | 64 | n.d. | n.d. | n.d. |
| 408 | 360,6 | 4,0 | 64 | 0,14 | 0,21 | 112,0 |

| | | | | | | |
|---|---|---|---|---|---|---|
| n.b.: nicht bestimmt | | | | | | |

Ein durchgängiges Absenken der Salzbadtemperatur führt zu einer Verschlechterung der Produktgaszusammensetzung.

### Beispiel 6 (nicht erfindungsgemäß):

Die unterschiedlichen Betriebsparameter sind in der folgenden Tabelle zusammengefasst:

| Laufzeit [Tage] | Salzbadtemperatur [°C] | Gasstrom volumen [Nm³/h] | Beladung [g o-Xylol/Nm³] | o-Xylol im Reaktoraustritt [Gew.-%] | Phthalid im Reaktoraustritt [Gew.-%] | PSA-Ausbeute [Gew.-%] |
|---|---|---|---|---|---|---|
| 192 | 370 | 4,0 | 64 | 0,08 | 0,25 | 111,5 |
| 216 | 368 | 4,0 | 67 | 0,11 | 0,26 | 112,2 |
| 240 | 366 | 4,0 | 70 | 0,12 | 0,28 | 112,1 |

Ein durchgängiges Absenken der Salzbadtemperatur (kombiniert mit einer Erhöhung der Beladung an o-Xylol) in den Beispielen 5 und 6 führen zu einer Verschlechterung der Produktgaszusammensetzung.

### Beispiel 7 (nicht erfindungsgemäß):

Die unterschiedlichen Betriebsparameter sind in der folgenden Tabelle zusammengefasst:

| Laufzeit [Tage] | Salzbadtemperatur [°C] | Gasstrom -volumen [Nm³/h] | Beladung [g o-Xylol/Nm³] | o-Xylol im Reaktoraustritt [Gew.-%] | Phthalid im Reaktoraustritt [Gew.-%] | PSA-Ausbeute [Gew.-%] |
|---|---|---|---|---|---|---|
| 168 | 368 | 4 | 58,5 | 0,08 | 0,21 | 113,0 |
| 192 | 366 | 4 | 60,5 | 0,11 | 0,23 | 113,0 |
| 216 | 364 | 4 | 60,5 | 0,15 | 0,28 | 113,8 |
| 240 | 364 | 4 | 55,5 | 0,12 | 0,24 | 114,0 |
| 264 | 364 | 3,5 | 60,5 | 0,04 | 0,11 | 112,5 |

Ein kontinuierliches Absenken führt auch bei identischer Beladung und Gasstromvolumen (Analysen nach 192 und 216 h) zu einer Verschlechterung der Produktgaszusammensetzung. Durch Reduktion der Beladung bzw. der Verweilzeit kann die Produktqualität auf Kosten der Produktivität verbessert werden (Analysen nach 240 h und nach 264 h).

### Beispiel B: Oxidation von Naphthalin bzw. Mischungen von Naphthalin und o-Xylol zu Phthalsäureanhydrid

### Herstellung der Katalysatorlagen

### Katalysatorlage 1 (KL1)

2 kg Steatitringe (Magnesiumsilikat) in Form von Ringen mit Abmessungen von 8 mm x 6 mm x 5 mm wurden in einer Fließbettapparatur mit 860 g einer Suspension aus 6,92 g Cäsiumcarbonat, 562,34 g Titandioxid (Fuji TA 100 C; Anatas, BET-Oberfläche 20 m²/g), 42,86 g Vanadiumpentoxid, 1.75 g Niobpentoxid, 1587,96 g demineralisiertem Wasser und 97,7 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450°C betrug die auf die Steatitringe aufgebrachte Aktivmasse 9,0 %. Die analysierte Zusammensetzung der Aktivmasse bestand aus 7 % V₂O₅, 0,2 % Nb₂O₅, 0,92 % Cs, Rest TiO₂.

Katalysatorlage 2 (KL2): Herstellung analog KL1 Variation der Zusammensetzung der Suspension. Nach Kalzination des Katalysators für eine Stunde bei 450 °C betrug die auf die Steatitringe aufgebrachte Aktivmasse 9,1 %. Die analysierte Zusammensetzung der Aktivmasse bestand aus 7 % V₂O₅, 0,2 % Nb₂O₅, 0,67 % Cs, Rest TiO₂ mit einer mittleren BET-Oberfläche von 20 m²/g (Fuji TA 100 C Anatas).

Katalysatorlage 3 (KL3): Herstellung analog KL1 Variation der Zusammensetzung der Suspension. Nach Kalzination des Katalysators für eine Stunde bei 450 °C betrug die auf die Steatitringe aufgebrachte Aktivmasse 9,0 %. Die analysierte Zusammensetzung der Aktivmasse bestand aus 7 % V₂O₅, 0,2 % Nb₂O₅, 0,35 % Cs, 0,04 % K (in die Suspension eingebracht als Sulfat), 0,03 % P (in die Suspension eingebracht als Dihydrogenphosphat) Rest TiO₂ mit einer mittleren BET-Oberfläche von 22,1 m²/g (Mischung aus Fuji TA 100 C Anatas BET-Oberfläche von 20 m²/g und Fuji TA 100 CT Anatas BET-Oberfläche von 27 m²/g).

Katalysatorlage 4 (KL4): Herstellung analog KL1 Variation der Zusammensetzung der Suspension. Nach Kalzination des Katalysators für eine Stunde bei 450 °C betrug die auf die Steatitringe aufgebrachte Aktivmasse 8,0 %. Die analysierte Zusammensetzung der Aktivmasse bestand aus 20 % V₂O₅, 0,18 % P (in die Suspension eingebracht als Dihydrogenphosphat), 0,24 % W (in die Suspension eingebracht als WO₃) Rest TiO₂ mit einer mittleren BET-Oberfläche von 20 m²/g (Fuji TA 100 C Anatas).

### Katalytische Oxidation von Naphthalin bzw. einer Mischung von Naphthalin und o-Xylol zu Phthalsäureanhydrid im Modellrohrmaßstab

Die katalytische Oxidation von o-Xylol zu Phthalsäureanhydrid wurde in einem salzbadgekühlten Rohrreaktor mit einem Innendurchmesser der Rohre von 25 mm durchgeführt. Von Reaktoreingang zu Reaktorausgang wurden jeweils 80 cm KL1, 80 cm KL2, 90 cm KL3 und 90 cm KL4 in ein 3,5 m langes Eisenrohr mit der lichten Weite von 25 mm eingefüllt. Das Eisenrohr war zur Temperaturregelung von einer Salzschmelze umgeben, eine 4 mm Außendurchmesser Thermohülse mit eingebautem Zugelement diente der Katalysatortemperaturmessung.

Präformierung der Katalysatoren erfolgte unter 0,1 Nm³/h Luft bei 400°C für ca. 24 Stunden.

Nach Anfahren des Katalysators bei 380°C wurde das Rohr stündlich von oben nach unten mit 3,0 bis 4,0 Nm³ Luft mit Beladungen an 97,5 Gew.-%igem Naphthalin bzw. Mischungen von 97,5 Gew.-%igem Naphthalin und 99-99,4 Gew.-%igem o-Xylol von in Summe 30 bis 80 g/Nm³ geleitet. Die Wärmebetttemperatur (Salzbadtemperatur) wurde ausgehend von 386°C abgesenkt. Die PSA-Ausbeuten wurden im Reaktionsaustrittsgas gemessen und sind in Massen-% (kg PSA pro kg umgesetztem Naphthalin bzw. Naphthalin und o-Xylol), bezogen auf 100 %-iges Edukt angeben.

### Beispiel 8 (erfindungsgemäß):

Die unterschiedlichen Betriebsparameter sind in der folgenden Tabelle zusammengefasst:

| Laufzeit [Tage] | Salzbadtemperatur [°C] | Gastromvolumen [Nm³/h] | Beladung [g/ Nm³] | | | Naphthochinon im Reaktoraustritt [Gew.-%] | Phthalid in Reaktoraustritt [Gew.-%] | PSA-Ausbeute [Gew.-%] |
|---|---|---|---|---|---|---|---|---|
| | | | Sum -me | davon Naphthalin | davon o-Xylol | | | |
| 96 | 380 | 4 | 40,1 | 40,1 | 0 | 1,86 | 0,00 | 105,6 |
| 120 | 377 | 4 | 40,3 | 40,3 | 0 | 1,95 | 0,00 | 105,8 |
| 192 | 377 | 4 | 45,4 | 40,4 | 5 | 1,58 | 0,01 | 107,5 |
| 240 | 376 | 4 | 45,4 | 40,4 | 5 | 1,71 | 0,01 | 105,7 |
| 264 | 376 | 4 | 45,4 | 40,4 | 5 | 1,61 | 0,01 | 106,0 |

Ein Aussetzen des Absenkes der Salzbadtemperatur führt zu einer Reduktion des Unteroxidationsproduktes Naphthochinon und damit zu einer verbesserten Produktgaszusammensetzung.

## Patentansprüche

1. Verfahren zur Herstellung von Carbonsäuren und/oder Carbonsäureanhydriden durch Gasphasenoxidation von aromatischen Kohlenwasserstoffen, bei dem man einen Gasstrom, der mindestens einen aromatischen Kohlenwasserstoff und molekularen Sauerstoff umfasst, kontinuierlich über einen durch ein Wärmeträgermedium thermostatisierten Katalysator leitet, **dadurch gekennzeichnet, dass** die Temperatur des Wärmeträgermediums während des Anfahrens des Reaktors von einer Temperatur im Bereich von 380 bis 410°C auf eine Temperatur im Bereich von 340 bis 365°C abgesenkt wird und dabei für mindestens 24 Stunden konstant gehalten wird mit einer Änderung von weniger als 0,1°C pro 24 Stunden und währenddessen weder die Beladung des Gasstroms mit Kohlenwasserstoffen noch das Gastromvolumen um mehr als 3 % erhöht werden.

2. Verfahren nach Anspruch 1, **dadurch gekennzeichnet, dass** die Temperatur des Wärmeträgermediums mindestens 48 Stunden konstant gehalten wird.

3. Verfahren nach einem der Ansprüche 1 oder 2, **dadurch gekennzeichnet, dass** in dem Zeitraum, während dessen die Temperatur des Wärmeträgermediums konstant gehalten wird, die Beladung des Gasstroms mit Kohlenwasserstoffen um maximal 1,5 % erhöht wird.

4. Verfahren nach einem der Ansprüche 1 oder 2, **dadurch gekennzeichnet, dass** in dem Zeitraum, während dessen die Temperatur des Wärmeträgermediums konstant gehalten wird, das Gasstromvolumen um maximal 2,5 % erhöht wird.

5. Verfahren nach einem der Ansprüche 1 oder 2, **dadurch gekennzeichnet, dass** die Beladung des Gasstroms mit Kohlenwasserstoffen im Laufe des Anfahrens von 25 bis 30 g/Nm³ auf 70 bis 120 g/Nm³ erhöht wird.

6. Verfahren nach einem der Ansprüche 1 bis 5, **dadurch gekennzeichnet, dass** Phthalsäureanhydrid aus o-Xylol und/oder Naphthalin hergestellt wird.

7. Verfahren nach einem der Ansprüche 1 bis 6, **dadurch gekennzeichnet, dass** die katalytische aktive Masse des Katalysators zumindest Vanadiumpentoxid und Titandioxid umfasst.

## Claims

1. A process for preparing carboxylic acids and/or carboxylic anhydrides by gas phase oxidation of aromatic hydrocarbons, in which a gas stream comprising at least one aromatic hydrocarbon and molecular oxygen is passed continuously over a catalyst thermostatted by a heat carrier medium, which comprises lowering the temperature of the heat carrier medium during the startup of the reactor from a temperature in the range from 380 to 410°C to a temperature in the range of 340 to 365°C and keeping it constant for at least 24 hours with a variation of less than 0.1°C per 24 hours, during which neither the loading of the gas stream with hydrocarbons nor the gas stream volume is increased by more than 3%.

2. The process according to claim 1, wherein the temperature of the heat carrier medium is kept constant for at least 48 hours.

3. The process according to either of claims 1 and 2, wherein the loading of the gas stream with hydrocarbons in the period during which the temperature of the heat carrier medium is kept constant is increased by a maximum of 1.5%.

4. The process according to either of claims 1 and 2, wherein the gas stream volume in the period during which the temperature of the heat carrier medium is kept constant is increased by a maximum of 2.5%.

5. The process according to either of claims 1 and 2, wherein the loading of the gas stream with hydrocarbons is increased in the course of startup from 25 to 30 g/m³ (STP) to 70 to 120 g/m³ (STP).

6. The process according to any of claims 1 to 5, wherein phthalic anhydride is prepared from o-xylene and/or naphthalene.

7. The process according to any of claims 1 to 6, wherein the catalytically active material of the catalyst comprises at least vanadium pentoxide and titanium dioxide.

## Revendications

1. Procédé de fabrication d'acides carboxyliques et/ou d'anhydrides d'acides carboxyliques par oxydation en phase gazeuse d'hydrocarbures aromatiques, selon lequel un courant gazeux, qui comprend au moins un hydrocarbure aromatique et de l'oxygène moléculaire, est acheminé en continu sur un catalyseur thermostaté par un milieu caloporteur, **caractérisé en ce que** la température du milieu caloporteur est abaissée pendant le démarrage du réacteur d'une température dans la plage allant de 380 à 410 °C à une température dans la plage allant de 340 à 365 °C, et maintenue constante pendant au moins 24 heures avec une modification de moins de 0,1 °C par 24 heures, et, pendant ce temps, ni le chargement du courant gazeux avec des hydrocarbures, ni le volume du courant gazeux ne sont augmentés de plus de 3 %.

2. Procédé selon la revendication 1, **caractérisé en ce que** la température du milieu caloporteur est maintenue constante pendant au moins 48 heures.

3. Procédé selon l'une quelconque des revendications 1 ou 2, **caractérisé en ce que** le chargement du courant gazeux avec des hydrocarbures est augmenté d'au plus 1,5 % durant la période pendant laquelle la température du milieu caloporteur est maintenue constante.

4. Procédé selon l'une quelconque des revendications 1 ou 2, **caractérisé en ce que** le volume du courant gazeux est augmenté d'au plus 2,5 % durant la période pendant laquelle la température du milieu caloporteur est maintenue constante.

5. Procédé selon l'une quelconque des revendications 1 ou 2, **caractérisé en ce que** le chargement du courant gazeux avec des hydrocarbures est augmenté au cours du démarrage de 25 à 30 g/Nm³ à 70 à 120 g/Nm³.

6. Procédé selon l'une quelconque des revendications 1 à 5, **caractérisé en ce que** de l'anhydride de l'acide phtalique est préparé à partir d'o-xylène et/ou de naphtaline.

7. Procédé selon l'une quelconque des revendications 1 à 6, **caractérisé en ce que** la masse catalytiquement active du catalyseur comprend au moins du pentoxyde de vanadium et du dioxyde de titane.
